# EUROPEAN PATENT APPLICATION

(11) **EP 1 323 445 A1**
(43) Date of publication of application: **02.07.2003**
(21) Application number: 02011810.5
(22) Date of filing: 28.05.2002
(51) Int. Cl.: A61M 5/32, A61B 17/20, A61B 17/06

(54) **Medical needle assemblies**

(30) Priority: 28.12.2001 US 344126 P; 09.05.2002 US 141174
(71) Applicant: Becton, Dickinson and Company, Franklin Lakes, New Jersey 07417 (US)
(72) Inventor: Swenson, John S., Wayne, NJ 07470 (US); Caizza, Richard James, Vernon, NJ 07462 (US); Swenson, Kirk D., North Caldwell, NJ 07006 (US); Prais, Alfred Wesley, Hewitt, NJ 07421 (US); Alchas, Paul G., Wayne, NJ 07470 (US)
(74) Representative: Selting, Günther, Dipl.-Ing.

(57) **Abstract**

A shieldable, single-use unit dose needle assembly for administering a unit dose of a vaccine includes a unit dose needle (12) attached to a base (40) with a sleeve (80) slidably attached thereto. The sleeve includes a guide slot (90) that extends through the tubular sleeve wall. The base includes a tab (50) disposed on an outer surface and extends through and beyond the guide slot. The unit dose needle includes a base end attached to the base and an opposed prong end (16) configured to hold a unit dose of vaccine. The needle assembly is configured such that movement of the tab (50) through the guide slot (90) enables an extension and a retraction movement of the needle (12) between an extended position with the needle extending from the distal end of the sleeve and a retracted position with the needle contained within the internal sleeve opening.

## Description

### CROSS-REFERENCE TO RELATED APPLICATIONS

This application claims priority from U.S. Provisional Patent Application Serial No. 60/344,126, filed December 28, 2001, entitled "Bifurcated Needle Assembly with Needle Shielding Provisions."

### BACKGROUND OF THE INVENTION

### 1. Field of the Invention

The present invention relates to needles for use in medical procedures and, in particular, to safety shielded needles and needle assemblies for use in medical procedures.

### 2. Description of Related Art

Bifurcated or forked end needles are well-known for providing a simple and effective means for a doctor to administer a vaccine. During use, the bifurcated tip of the bifurcated needle is put into contact with either a dried or liquid substance, which adheres to the bifurcated needle tip. The bifurcated needle tip is then put into contact with the skin of the patient who is being administered the vaccination. The skin is either scratched or pierced with the needle tip so that the vaccination material may be absorbed into the skin of the patient. An alternative method of delivering the vaccination includes placing a drop of the vaccine onto the skin of the patient and contacting the skin of the patient with the bifurcated needle tip through the drop of vaccination. Alternatively, although not ideal, a standard pointed needle tip may also be used when the drop of vaccination is applied directly to the skin of the patient.

The bifurcated needle is considered a significant medical advancement because it has allowed more people to be vaccinated with less serum. This has been especially important for those living in less developed areas because of the efficient and easy to use design, as well as the ease of replication.

Vaccination effectiveness, however, is reduced if the bifurcated needle is reused too many times. Moreover, reuse of such vaccination needles exposes patients to the risk of transmission of infectious diseases through percutaneous contact through the skin. Additionally, medical care workers using traditional vaccination needles are at an increased risk of exposure to infectious diseases due to the design of such needles, which makes them difficult to handle, as well as due to the repeated use of such needles.

In particular, bifurcated needles used to administer vaccinations are not traditionally sterilized or packaged in a single-use container that would enable convenient storage and subsequent use. Additionally, such needles have traditionally been difficult to handle in that they typically do not include a hub attached to the opposite end of a needle from the tip, and do not typically include any sort of shield for protection from the needle prior to and during use.

For example, U.S. Patent No. 3,194,237 to Rubin discloses a vaccination needle having a main shank with a pair of prongs at one end that define a slot of predetermined length, width, and depth therebetween to hold an amount of liquid by capillary action. The shank of the needle is of sufficient length so that the non-prong end will function as a handle. U.S. Patent No. 3,948,261 to Steiner discloses a reusable unit dose container for vaccines contained within a rigid receptacle, with a compressible closure for supporting a bifurcated needle bearing dried vaccine. The closure is adapted to support the needle in the container during a lyophilizing process while liquid vaccine is dried on the needle. The closure has grooves which permit the vaporized liquid from the vaccine to be withdrawn from the receptacle during lyophilizing, and can further seal the container.

Numerous devices have been developed in the medical field for shielding needles after use. Many of these devices are somewhat complex and costly. In addition, many of these devices are cumbersome to use in performing procedures. Furthermore, some of the devices are so specific that they preclude use of the device in certain procedures or with certain devices and/or assemblies.

For example, U.S. Patent No. 6,093,170 to Hsu et al. discloses a safety syringe that includes a barrel having two trapezoidal-shaped tabs, a sleeve with a guide slot and limit slots extending from the circumferential surface at the two ends of the guide slot, and an insertion section formed at the inside end of the limit slot. The tabs are utilized to engage and the insertion section enables the positioning of the barrel and the sleeve. U.S. Patent No. 5,591,138 to Vaillancourt discloses a multicomponent needle assembly that includes a V-shaped slot provided at the rear of a guide slot so that a retractable sheath can be locked in a retracted position. A cam surface is provided at the front of the guide slot to direct the projection on the sheath into a position to lock of the sheath in an extended position.

In view of the foregoing, there exists a need for a safety assembly for use with a unit dose vaccination needle that is easily manufactured, that is simple to use, that is easily sterilized and maintained in a sterile condition until used, that can be safely disposed of, and that does not interfere with normal practices of bifurcated needle use.

### SUMMARY OF THE INVENTION

The present invention is directed to a shieldable, single-use needle assembly for administering a unit dose of a vaccine. The assembly includes a unit dose needle attached to a base, with a sleeve slidably attached thereto. The sleeve includes a tubular wall defining an internal sleeve opening that extends between a proximal end and an opposed distal end of the sleeve. The sleeve further includes a guide slot that extends through the tubular wall and into the internal sleeve opening. The guide slot includes a longitudinal extent extending longitudinally along the sleeve, a proximal limit extent disposed at a first end of the guide slot adjacent to the proximal end of the tubular wall, and a distal limit extent disposed at a second end of the guide slot adjacent to the distal end of the tubular wall. The proximal and distal limit extents extend along a circumferential surface of the tubular wall. The base includes a tab disposed on an outer surface thereof. The base is contained within the internal sleeve opening with the tab extending through and beyond the guide slot. The unit dose needle includes a base end attached to the base and an opposed prong end configured to hold a unit dose of a vaccine. The needle assembly is configured such that movement of the tab through the guide slot between the proximal limit extent and the distal limit extent provides a guiding action that enables an extension and a retraction movement of the needle between an extended position, with the needle extending from the distal end of the sleeve, and a retracted position, with the needle contained within the internal sleeve opening. The proximal and distal limit extents fix the position of the needle between the extended position and the retracted position.

In a further embodiment, the present invention is directed to a shieldable, single-use needle assembly for administering a unit dose of a vaccine, which includes a base, a unit dose needle having a base end attached to the base and an opposed prong end configured to hold a unit dose of a vaccine, a sleeve including a tubular wall defining an internal sleeve opening extending between a proximal end and an opposed distal end, and a guiding element interengaged between the sleeve and the base. The sleeve coacts axially with the base between a prong exposed position and a prong encapsulated position, and the guiding element provides limited axial movement of the sleeve with respect to the base. The guiding element may be a tab extending from the base into a slot in the sleeve, and the slot may extend through the sleeve, with the tab acting as a means to manually move the base and unit dose needle into the sleeve. Also, the base may include a handle extension extending beyond the proximal end of the sleeve.

In another embodiment, the present invention includes a shieldable, single-use needle assembly for administering a unit dose of a vaccine including a base, a unit dose needle, a tip guard, and a tether element. The unit dose needle includes an axis, a base end attached to the base and an opposed prong end configured to hold a unit dose of a vaccine. The tip guard is slidable along the unit dose needle axis from a first position adjacent the base to a second position encapsulating the unit dose needle prong end. The tether element has a base end integral with the base, a guard end integral with the tip guard, and a hinge between the base end and the guard end. The tether element further includes a forward member delimited by the guard end and the hinge which is flexibly pivotable with respect to the guard, a rearward end delimited by the base end and the hinge which is flexibly pivotable with respect to the base. The tether element has an angle between the forward and rearward members, wherein forward movement of the hinge enlarges the angle and provides direct forward movement of the guard with respect to the base.

### BRIEF DESCRIPTION OF THE DRAWINGS

FIG. 1 is an exploded perspective view of a shieldable single-use needle assembly of the present invention;

FIG. 2 is a perspective view of the shieldable needle assembly of FIG. 1 shown with the needle in a retracted position;

FIG. 3 is a perspective view of the shieldable needle assembly of FIG. 1 shown with the needle in an extended position;

FIG. 4 is a side-sectional view of the shieldable needle assembly as shown in FIG. 2 with the needle in a retracted position;

FIG. 5 is a side-sectional view of the shieldable needle assembly as shown in FIG. 3 with the needle in an extended position;

FIG. 6 is an exploded cross-sectional view of a unit dose needle and a base in an alternate embodiment of the present invention;

FIG. 7 is a perspective view of a needle and a base in a further embodiment of the present invention;

FIG. 8 is a side-sectional view of a needle and a base with a handle extension in a further embodiment;

FIG. 9 is a perspective view of a shieldable needle assembly in a further embodiment of the present invention including ribs on the shield;

FIG. 10 is a side-sectional view of a shieldable needle assembly in yet a further embodiment of the present invention with the needle in an extended position;

FIG. 11 is a side-sectional view of the shieldable needle assembly of FIG. 10 with the needle in a retracted position;

FIG. 12 is a side-sectional view of a shieldable needle assembly in a further embodiment of the present invention including a release latch;

FIG. 13 is a perspective view of a shieldable needle assembly in accordance with a further embodiment of the present invention including a telescoping needle clip; and

FIG. 14 is a cross-sectional view of the assembly of FIG. 13.

### DETAILED DESCRIPTION

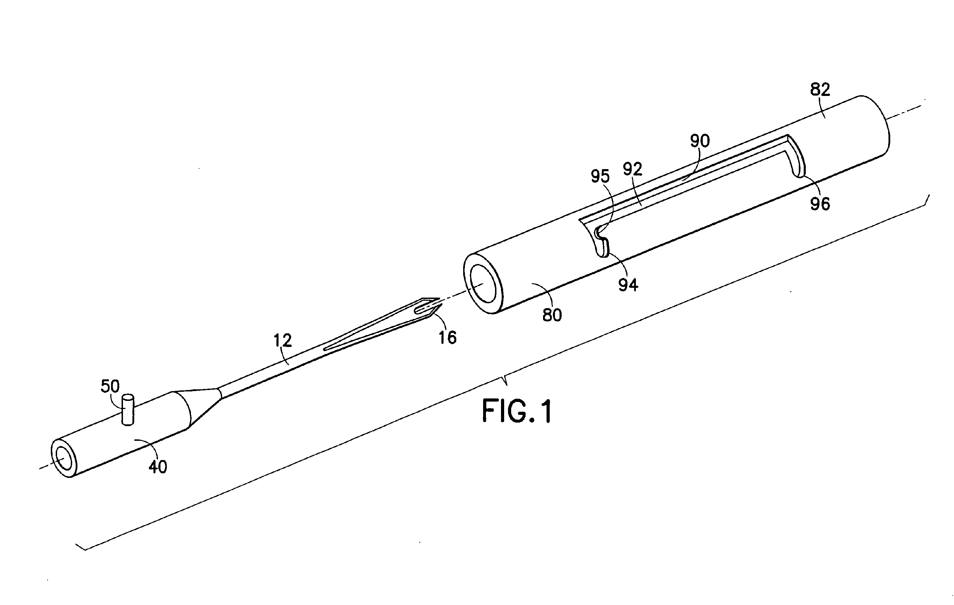

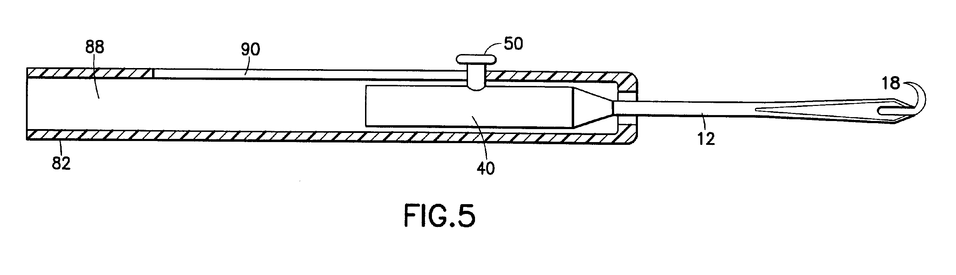

Referring to the drawings in which like reference characters refer to like parts throughout the several views thereof, FIGS. 1-5 depict a shieldable single-use unit dose needle assembly **10** in accordance with the present invention and the related features. The unit dose needle assembly **10** is intended for use for the administration of vaccines applied to or through the skin of the patient, and is intended as a single-use vaccination needle assembly including features to maintain sterility of the needle during packaging and to provide ease of use for the medical practitioner, as will be described in more detail herein.

The needle assembly **10** generally includes an external shield in the form of a tubular hollow sleeve **80,** with a base **40** extending within the sleeve **80**, and with a unit dose needle **12** extending from the base **40.** As will be described in more detail herein, the sleeve **80** and the base **40** are axially movable or displaceable with respect to each other, thereby causing the unit dose needle **12** to move between a retracted position contained entirely within sleeve **80** and an extended position, extending from sleeve **80**.

More particularly, as shown in FIGS. 1 and 3-5, the needle assembly **10** includes a unit dose needle for administering a unit dose of a vaccine, such as unit dose needle **12.** While needle assembly **10** is described herein in terms of a preferred embodiment including a unit dose needle **12** as the unit dose needle, needle assembly **10** may include any unit dose needle capable of administering a unit dose of a vaccine, such as in a dry powder or liquid form, as is well-known in the art.

The unit dose needle **12** includes a proximal end **14**, and an opposed distal end **16.** Unit dose needle **12** is provided with two sharp prongs **18** positioned at an opposed distal end **16** of the needle. The prongs **18** are separated by a U-shaped or V-shaped channel **20** configured to hold a unit dose of vaccine. The prongs **18** are intended to penetrate or abrade the skin of the patient to administer the vaccine disposed in the U-shaped channel **20.** Unit dose needle **12** may be constructed of any material known in the art, such as metal or plastic, and is desirably constructed of a medical grade surgical steel.

The vaccine may be in any suitable physical form. Suitable physical forms for the vaccine include, but are not limited to, liquids, such as solutions, emulsions, and dispersions, or dry powders. Typically, the vaccine will be in a liquid form. Moreover, the vaccine is desirably associated with needle assembly **10** during storage, and may therefore be contained within the U-shaped channel **20** during storage. Alternatively, the vaccine may be provided as a separate component, with unit dose needle **12** being contacted with the vaccine after removing needle assembly **10** from its associated packaging just prior to use of the needle for administration of the vaccine.

Needle assembly **10** further includes a base **40,** which may be integrally formed with unit dose needle **12,** or separately attached thereto. Base **40** includes a generally cylindrical housing **42** extending from a proximal end **44** to a distal end **46.** Base **40** further includes a guide element such as a tab **50** disposed on an outer surface of the housing **42** of base **40**. Tab **50** may be integral with housing **42** or alternately may be separately attached such as by a threaded screw means.

Needle assembly **10** further includes sleeve **80** extending about base **40** in a concentrical manner, thereby enclosing and containing base **40**. In particular, sleeve **80** includes a generally tubular wall forming a hollow housing **82** extending between a proximal end **84** and an opposed distal end **86** and defining an internal sleeve opening **88** extending therethrough. Sleeve **80** further includes a guide slot **90** extending through the wall of housing **82** into the internal sleeve opening **88.** The guide slot **90** includes a longitudinal extent **92** extending longitudinally along the housing **82** of sleeve **80,** a proximal limit extent **94** disposed at a first end of the guide slot **90** adjacent the proximal end **84** of the sleeve **80**, and a distal limit extent **96** disposed at a second end of the guide slot **90** adjacent the distal end **86** of the sleeve **80.** The proximal and the distal limit extents **94, 96** extend circumferentially along a partial circumferential surface of the housing **82,** as shown in FIG. 1.

Sleeve **80** provides protection to personnel from needle sticks prior to movement of unit dose needle **12** between the retracted position and the extended position, as well as after unit dose needle **12** has been moved to the retracted position after use thereof.

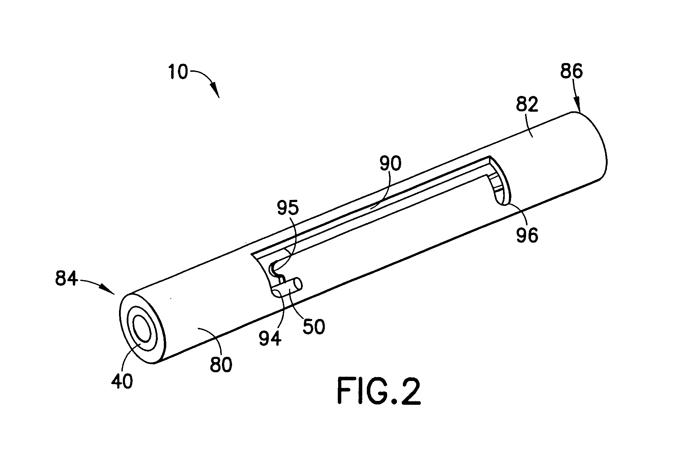

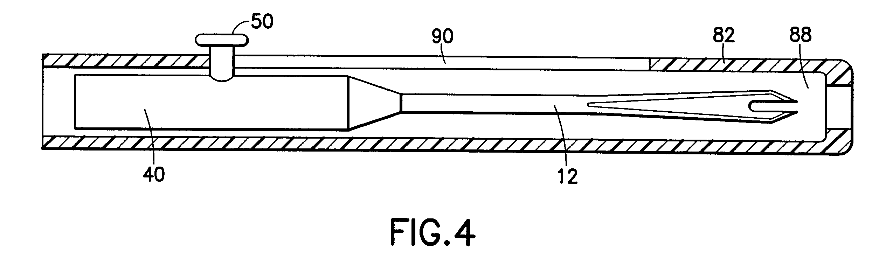

As noted, base **40** is contained within the internal sleeve opening **88** of sleeve **80,** when the unit dose needle is in the retracted position or the extended position. For example, as shown in FIGS. 2 and 4, the overall length of the base **40** and unit dose needle **12** extending therefrom is desirably slightly less than the overall length of sleeve **80,** such that base **40** and unit dose needle **12** are maintained entirely within internal sleeve opening **88** of sleeve **80** when unit dose needle **12** is retracted within sleeve **80.** Also, when the unit dose needle **12** is in the extended position, base **40** is contained entirely within sleeve **80,** with no portion of base **40** exposed from proximal end **84** of sleeve **80.** With base **40** extending entirely within sleeve **80,** there is no portion of base **40** for a user to grasp onto in order to axially move base **40** within sleeve **80** to expose unit dose needle **12** to the extended position or to retract unit dose needle **12** to the retracted position. However, as shown in FIGS. 2-5, tab **50** of base **40** extends through and beyond the guide slot **90** of sleeve **80.** As such, tab **50** can be moved through the guide slot **90** to effect movement of base **40** and, therefore, corresponding movement of unit dose needle **12**.

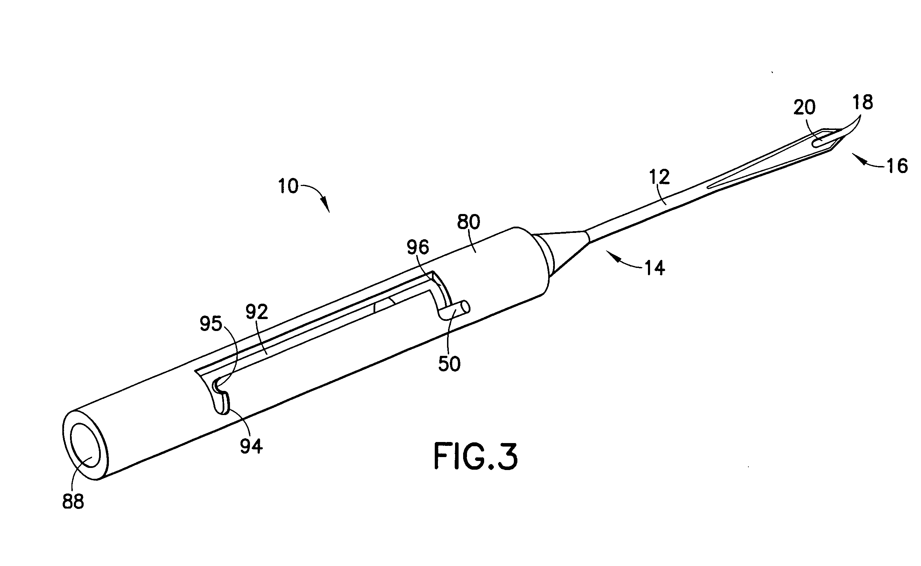

More particularly, a user can grasp needle assembly **10** between a thumb and finger at opposing sides of housing **82** of sleeve **80.** Since tab **50** extends through and beyond guide slot **90,** the user's index finger can be used to manipulate tab **50.** Tab **50** is capable of moving through the guide slot **90** between the proximal limit extent **94** and the distal limit extent **96,** which provides a guiding action that enables an extension and a retraction movement of the needle between an extended position as shown in FIG. 3, with the unit dose needle **12** extending from the distal end **86** of the sleeve **80**, and a retracted position as shown in FIG. 2, with the unit dose needle **12** contained within the internal sleeve opening **88.** As such, unit dose needle **12** can be moved between the extended position and the retracted position with one handed operation of needle assembly **10.**

The proximal limit extent **94** and the distal limit extent **96** fix the position of the unit dose needle **12** between the extended position and the retracted position. Desirably, a locking mechanism is provided, such as a lip **95** extending along a portion of the tubular wall of housing **82** within proximal limit extent **94** and distal limit extend **96,** thereby fixing the position of the tab within guide slot **90,** and providing a tactile feel to the user that the tab **50** has been moved within the guide slot **90** between proximal limit extent **94** and distal limit extend **96.**

It is further contemplated that the arrangement of the tab and the slot can be reversed, such that the sleeve **80** includes a tab, and the base **40** includes a guide slot for engagement therewith. As such, the tab of the sleeve **80** can slide within the guide slot of the base **40** in a similar manner as discussed above.

In use, needle assembly **10** may be packaged in sterile packaging, and is removed from the packaging for use. Either a film-based blister package may be used or alternatively a rigid hardpack type of packaging involving thermoplastic shields may be utilized. Needle assembly **10** may be provided as shown in FIG. 2, including unit dose needle **12** retracted within sleeve **80.** To prepare for use, unit dose needle **12** is placed in the extended position, by grasping sleeve **80** at opposing sides thereof, and by manipulating tab **50** through guide slot **90,** from proximal limit extend **94**, along longitudinal extent **92,** to distal limit extent **96,** to extend unit dose needle **12** from sleeve **80.** Alternatively, needle assembly **10** may be provided as depicted in FIG. 3, with unit dose needle **12** extending from sleeve **80,** and may further include a separate packaging cover (not shown) covering the unit dose needle **12.**

With unit dose needle **12** extending from sleeve **80,** needle assembly **10** can be used to administer an appropriate vaccine. For example, a unit dose of a vaccine contained within U-shaped channel **20** may be administered percutaneously to the patient by way of unit dose needle **12.** The unit dose of the vaccine may be contained within U-shaped channel **12** during packaging, or the unit dose of the vaccine may be placed within U-shaped channel **20** immediately prior to administration. The vaccination may be administered through several applications to the patient with the same assembly.

After administration of the vaccine is complete, the user grasps sleeve **80** at opposing sides thereof, and manipulates tab **50** through guide slot **90** in reverse manner, from distal limit extent **96,** along longitudinal extent **92**, to proximal limit extend **94,** thereby retracting unit dose needle **12** within sleeve **80.** Needle assembly **10** is safely shielded, and can be properly disposed of.

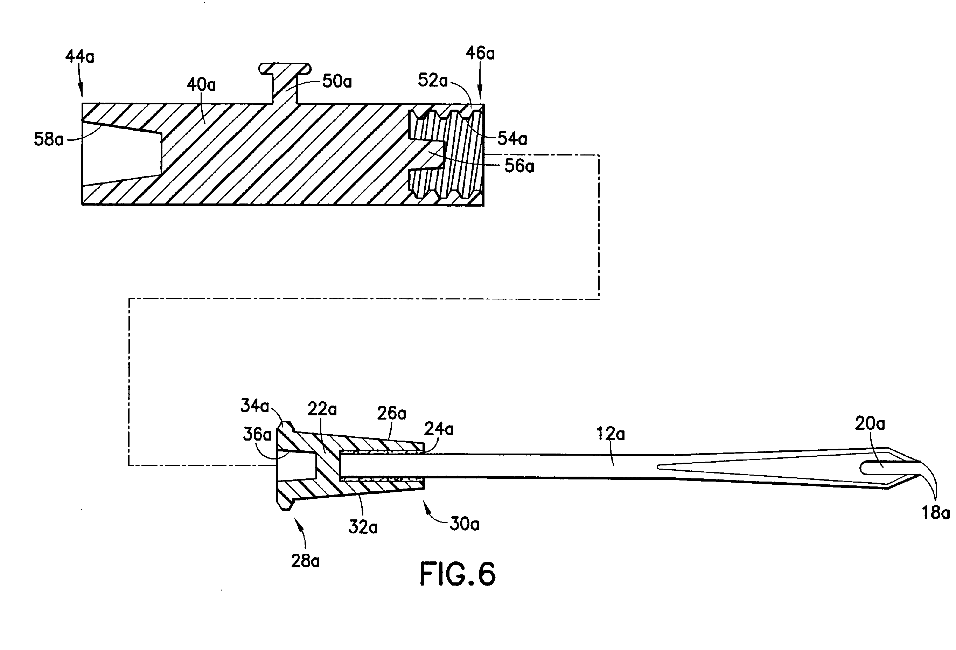

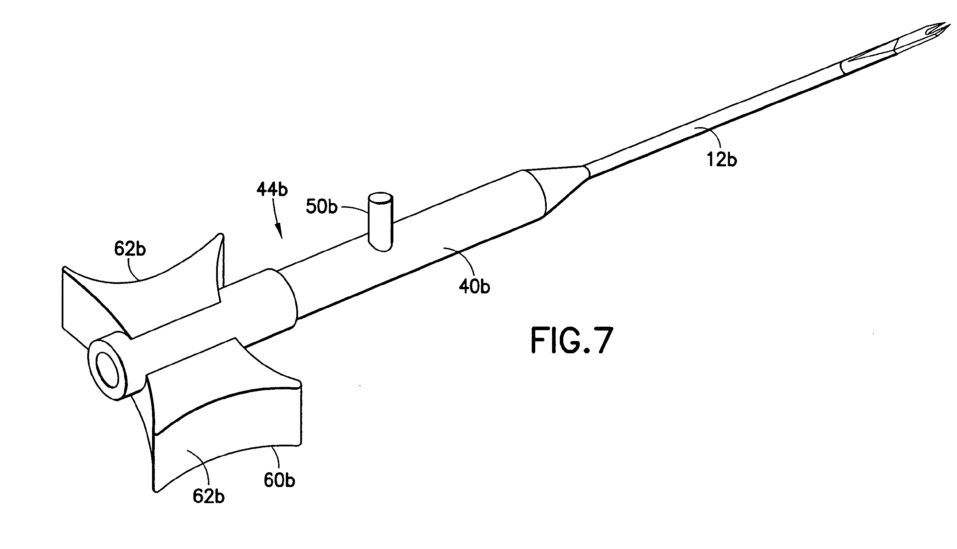

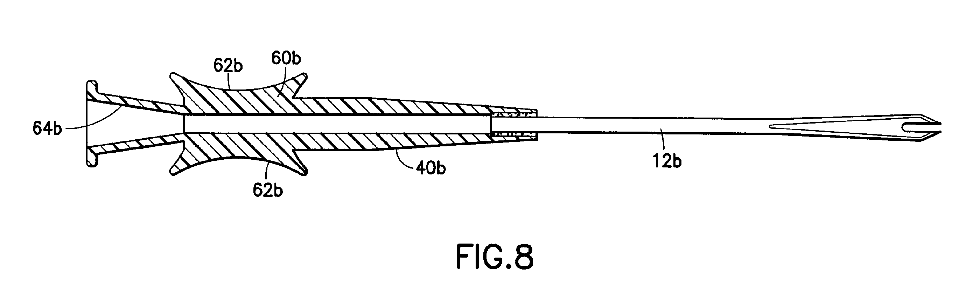

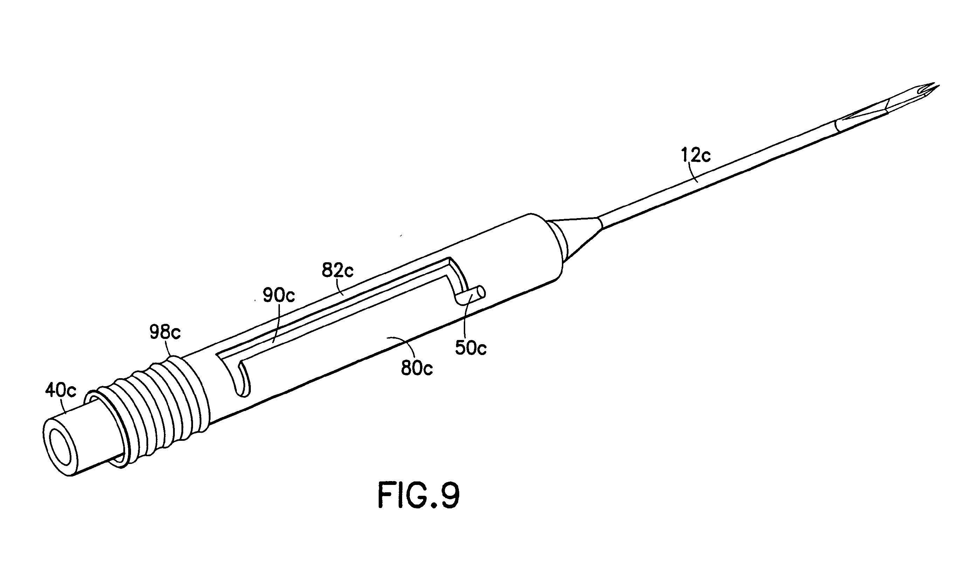

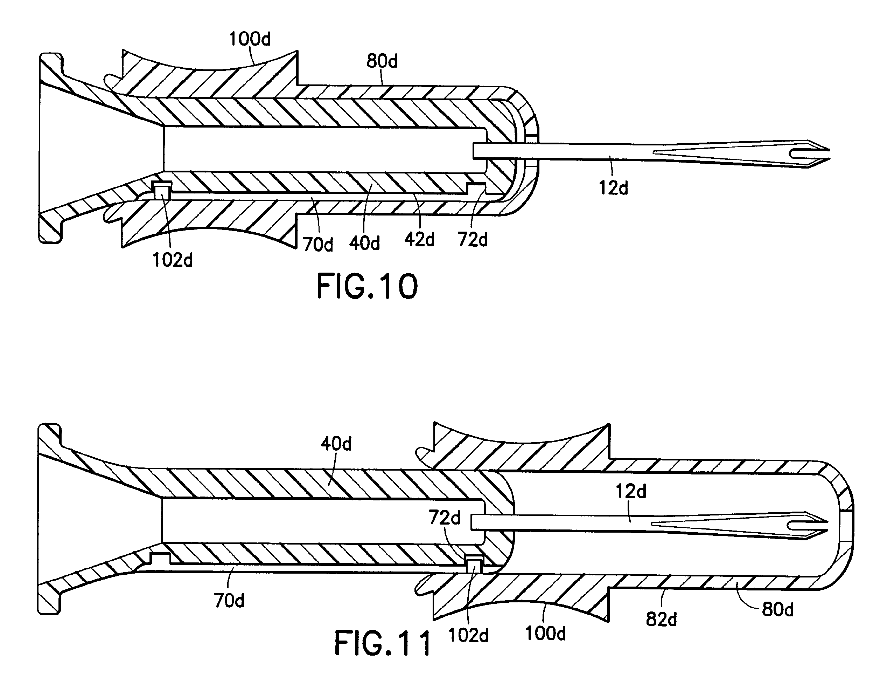

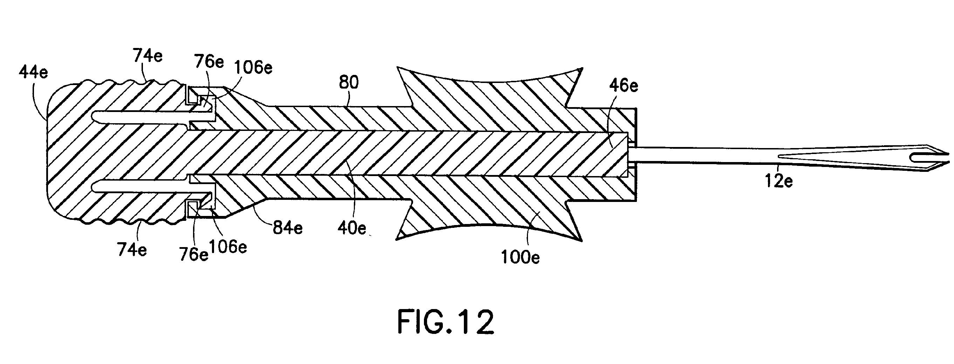

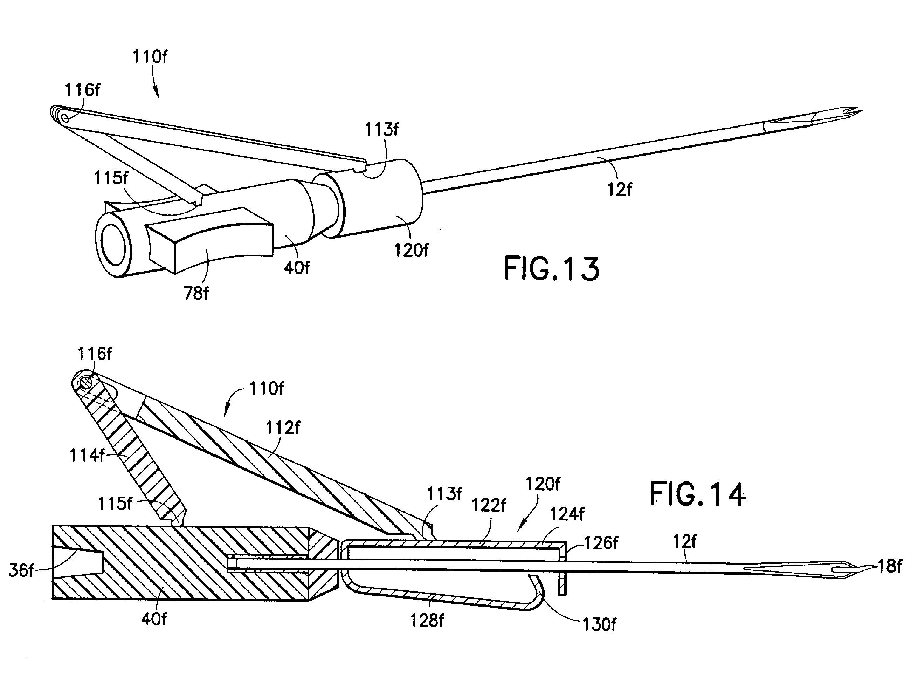

FIGS. 6-14 depict further embodiments of the present invention that include many components which are substantially identical to the components of FIGS. 1-5. Accordingly, similar components performing similar functions will be numbered identically to those components of FIGS. 1-5, except that a suffix "a" will be used to identify those similar components in FIG. 6; a suffix "b" will be used to identify those similar components in FIGS. 7 and 8; a suffix "c" will be used to identify those similar components in FIG. 9; a suffix "d" will be used to identify those similar components in FIGS. 10 and 11; a suffix "e" will be used to identify those similar components in FIG. 12; and a suffix "f" will be used to identify those similar components in FIGS. 13 and 14.

In the alternate embodiment shown in FIG. 6, unit dose needle **12a** may further include a hub **22a** fixedly attached to the proximal end **14a** of unit dose needle **12a,** such as through an adhesive joint **24a.** Adhesive joint **24a** may be provided through any adhesive capable of fixedly attaching or adhering unit dose needle **12a** to hub **22a,** such as an epoxy or equivalent adhesive. Hub **22a** includes a hub housing **26a** including a proximal end **28a** and a distal end **30a.** Desirably, distal end **30a** of hub **22a** includes an internal bore having an internal diameter of approximately the same size as or a slightly larger size than the outer diameter of the proximal end **14a** of unit dose needle **12a,** for accommodating and fixedly adhering unit dose needle **12a** within such an internal bore of hub **22a.**

The hub **22a** may further include a luer lug **34a** extending circumferentially about the proximal end thereof, with an internal luer taper **36a** which extends internally within a portion of hub housing **26a.** Internal luer taper **36a** is designed to engage a separate member, such as a medical device in the form of a hypodermic syringe or standard needle holder, or a handle portion, as described in more detail with respect to the embodiment of FIG. 7 described herein. Threads (not shown) may further be provided within internal luer taper **36a,** for establishing threaded engagement with a corresponding threaded surface of such a separate member.

In the embodiment of FIG. 6, base **40a** further includes a forward annular skirt **52a** extending from distal end **46a** of base **40a.** Annular skirt **52a** includes a tapered nub **56a** having a profile for mating with the internal surface of internal luer taper **36a** of hub **22a.** In addition, annular skirt **52a** includes internal threads **54a** extending within annular skirt **52a** on an internal surface thereof. Internal threads **54a** of annular skirt **52a** mate with luer lug **34a** of hub **22a**, thereby providing interengaging threaded structure between base **40a** and hub **22a,** for attaching unit dose needle **12a** to base **40a.** Alternatively, a system of luer locks may be used to effect mating between base **40a** and hub **22a.** Further, base **40a** may include an internal luer taper **58a** at the proximal end thereof, for further mating with a separate member such as a medical device or a handle assembly.

In such an embodiment including unit dose needle **12a** and base **40a** attached through hub **22a,** the sleeve is provided in a similar manner as described above. A needle cover (not shown) may further be provided, which may engage tapered surface **32a** of hub **22a,** providing a sterile packaging cover for unit dose needle **12a** during shipping and storage.

Such an embodiment enables unit does needle **12a** and hub **22a** to be provided as a separate member in the form of a unit dose assembly. Such a unit dose assembly can be mated with a separate shield assembly, including a base **40a** with a sleeve extending thereabout, as described hereinabove. The unit dose assembly and the shield assembly are desirably attached, such as internal luer taper **36a** and annular skirt **52a.** In this manner, the unit dose needle assembly including unit dose needle **12a** and hub **22a** can be separately packaged as a sterile assembly (which may include a removable needle packaging cover,) and can be mated with the separate shield assembly immediately prior to use.

In another embodiment depicted in FIGS. 7 and 8, a base **40b** includes a handle extension **60b,** extending from a proximal end **44b** of the base **40b.** Handle extension **60b** is designed to extend beyond the proximal end of the sleeve when a sleeve is provided around base **40b.** As such, handle extension **60b** provides base **40b** with a surface for grasping by the user, thereby allowing the user to hold the base **40b** in one hand and manipulate the sleeve with the other hand, to axially displace the sleeve with respect to base **40b** and thereby shield or unshield unit dose needle **12b.** Handle extension **60b** can also function as a handle to facilitate administering a vaccine with unit dose needle **12b.**

Handle extension **60b** may be integrally formed with base **40b,** or may be a separate member which is attached to the proximal end **44b** of base **40b,** such as through an internal luer taper as discussed with reference to FIG. 6. The handle extension **60b** may include a profile for accommodating a user's fingers. For example, handle extension **60b** may include one or more arcuate surfaces **62b** desirably extending along opposing sides thereof, for grasping between a user's finger and thumb. Moreover, handle extension **60b** may include an internal luer taper **64b,** for further mating with a separate member such as a medical device.

In a further embodiment of the invention, the sleeve may further include a profile for accommodating a user's fingers. For example, as shown in FIG. 9, sleeve **80c** may include a ribbed surface **98c** formed through a plurality of ribs oriented circumferentially about housing **82c** of sleeve **80c,** which may extend over a portion of housing **82c** or over the entire length of housing **82c.** Ribbed surface **98c** facilitates handling of the needle assembly by a user, whereby sleeve **80c** can be manipulated or rotated about base **40c** and moved axially with respect thereto through the guiding action of guide tab **50c** within guide slot **90c,** to place the needle assembly in an extended position or a retracted position. Although ribbed surface **98c** is shown, other types of ergonomically acceptable surfaces to accommodate a user's fingers may be used, such as one or more arcuate surfaces, indentations, or bumps to facilitate gripping of the sleeve. Moreover, in such an embodiment, sleeve **80c** can function as both a protective shield for unit dose needle **12c** and as a handle to facilitate administering a vaccine with unit dose needle **12c**.

FIGS. 10 and 11 depict a further embodiment of the present invention, in which the sleeve **80d** includes a tab **102d** extending preferably from an internal surface of the tubular housing **82d**, for interengagement within a slot **70d** extending along a portion of the outer surface of the housing **42d** of base **40d**. The slot **70d** extends longitudinally along at least a portion of the length of the outer surface of housing **42d** of base **40d.** The sleeve **80d** is capable of being slidably movable with respect to the base **40d** and unit dose needle **12d** to a position in which unit dose needle **12d** is shielded, as shown in FIG. 11. During such slidable movement, tab **102d** is guided within the slot **70d,** thereby providing a guiding action that enables the unit dose needle **12d** to be exposed and shielded between an extended position with the unit dose needle **12d** extending from sleeve **80d,** as shown in FIG. 10, and a retracted position with the unit dose needle **12d** contained within the sleeve **80d,** as shown in FIG. 11. A recessed pocket **72d** may further be provided at a portion of the slot **70d** adjacent the distal end of base **40d,** which provides a means for locking the sleeve **80d** in a position with the unit dose needle **12d** retracted and shielded within sleeve **80d.**

In such an embodiment, sleeve **80d** may further include a profile for accommodating a user's fingers, such as one or more arcuate surfaces **100d**.

In a further embodiment shown in FIG. 12, the base **40e** includes latches **74e** extending longitudinally along housing **42e** from proximal end **44e** toward distal end **46e,** forming a clip end **76e.** Such latches **74e** are provided for releasable engagement with sleeve **80e,** such as through detents **106e** at proximal end **84e** of sleeve **80e.** The latches **74e** engage the sleeve **80e** in such a way as to retain the sleeve **80e** in a first position with the unit dose needle **12e** in the extended position. The latches **74e** can be flexed to release the sleeve **80e,** enabling the sleeve **80e** to be moved to a position covering unit dose needle **12e.** A biasing member such as a spring (not shown) may also be provided to automatically propel sleeve **80e** to a position encompassing unit dose needle **12e** upon release of the engagement between latches **74e** and detents **106e.** A compression spring (not shown) can be used to accomplish this function.

A further embodiment of the present invention is depicted in FIGS. 13 and 14, which includes a tip guard assembly **120f** connected to base **40f** through a flexible tether mechanism **110f**. Tip guard assembly **120f** may be provided as a one-component tip guard assembly, as depicted in FIGS. 13-14, or may be provided as a multiple-component assembly including a separate flexible tip guard attached within a housing. Tip guard assembly **120f** including a housing **122f** forming a top extent **124f** defining the top portion of the tip guard assembly **120f** and extending longitudinally along a portion of unit dose needle cannula **12f.** Top extent **124f** bends downwardly at a forward end to form front end wall **126f.** At the rearward end, top extent **124f** bends backward to form spring leg **128f** which extends toward the front end wall **126f** of tip guard assembly **120f,** with lock out leg **130f** bending upward and backward to form an end wall, as seen in FIG. 14.

Tip guard assembly **120f** is interconnected with base **40f** through tether mechanism **110f**. Tether mechanism **110f** includes a forward arm **112f** and a rearward arm **114f.** Forward arm **112f** is attached to top extent **124f** of tip guard assembly **120f** and extends toward the rearward end of the needle assembly. Rearward arm **114f** is attached to the top surface of housing **42f** of base **40f,** and also extends toward the rearward end of the needle assembly. Forward arm **112f** and rearward arm **114f** meet and are interengaged at pivot hinge **116f,** providing a pivoting point therebetween. Additionally, forward arm **112f** and rearward arm **114f** are pivotally attached to tip guard assembly **120f** and base **40f** respectively such as through living hinge members **113f** and **115f** respectively. Forward living hinge member **113f** connecting forward arm **112f** to tip guard assembly **120f** is preferably more rigid than rearward hinge member **115f** such that movement of pivot hinge **116f** by forward manual user exertion causes tip guard assembly **120f** to move forward.

Tip guard assembly **120f** is slidably movable in a telescoping fashion along unit dose needle **12f** between a first position adjacent base **40f,** and a second position encompassing prongs **18f** of unit dose needle **12f** through tether mechanism **110f**. In particular, pivoting movement of forward arm **112f** and rearward arm **114f** about pivot hinge **116f,** such as by forward pressure exerted thereon, causes a pivoting movement at pivot hinge **116f**. This causes forward arm **112f** to extend, thereby telescoping tip guard assembly **120f** along unit dose needle **12f** toward prongs **18f**. During such movement, tip guard assembly **12f** slides or glides along unit dose needle **12f**, with lock out leg **130f** passing distally beyond prongs **18f**, and with the inherent resiliency of spring leg **128f** urging lock out leg **130f** over prongs **18f,** preventing a return movement, and thus preventing re-exposure of prongs **18f.**

Base **40f** may further include handles **78f** extending along opposing sides thereof, for facilitating holding of the assembly. In addition, base **40f** may include an internal luer taper **36f** for attachment to a separate medical device for providing further support for the user, as described above.

The sleeve, base, and hub of the assembly of the present invention may be constructed of any material, and are desirably constructed of a moldable plastic material. Suitable moldable plastics include, but are not limited to, polyethylenes, polypropylenes, polyamides, polyesters, and fluorinated polyethylenes.

While the present invention is satisfied by embodiments in many different forms, there is shown in the drawings and described herein in detail, the preferred embodiments of the invention, with the understanding that the present disclosure is to be considered as exemplary of the principles of the invention and is not intended to limit the invention to the embodiments illustrated. Various other embodiments will be apparent to and readily made by those skilled in the art without departing from the scope and spirit of the invention. The scope of the invention will be measured by the appended claims and their equivalents.

## Claims

1. A shieldable, single-use needle assembly for administering a unit dose of a vaccine comprising:
(a) a sleeve including a tubular wall defining an internal sleeve opening extending between a proximal end and an opposed distal end, said sleeve including a guide slot extending through the tubular wall into the internal sleeve opening, said guide slot including a longitudinal extent extending longitudinally along the sleeve, a proximal limit extent disposed at a first end of the guide slot adjacent said proximal end of said tubular wall and a distal limit extent disposed at a second end of the guide slot adjacent said distal end of said tubular wall, said proximal and distal limit extents extending along a circumferential surface of the tubular wall;
(b) a base including a tab disposed on an outer surface thereof, said base contained within said internal sleeve opening with said tab extending through and beyond said guide slot; and
(c) a unit dose needle having a base end attached to the base and an opposed prong end configured to hold a unit dose of a vaccine;
wherein movement of the tab through the guide slot between the proximal limit extent and the distal limit extent provides a guiding action that enables an extension and a retraction movement of the needle between an extended position with the needle extending from the distal end of the sleeve and a retracted position with the needle contained within the internal sleeve opening, and wherein the proximal and distal limit extents fix the position of the needle between the extended position and the retracted position.

2. The needle assembly of claim 1, wherein the unit dose needle comprises a bifurcated needle, wherein the prong end includes two pointed prongs which are capable of penetrating or abrading the skin of a patient, and wherein the prongs are separated by a U-shaped channel capable of holding the unit dose of a vaccine.

3. The needle assembly of claim 1, wherein the unit dose of a vaccine is a liquid.

4. The needle assembly of claim 1, wherein the base includes a handle extension extending beyond the sleeve from an end of the base opposite the unit dose needle.

5. The needle assembly of claim 4, wherein the handle extension includes an internal luer taper to engage a medical device.

6. The needle assembly of claim 4, wherein the handle extension includes a profile for accommodating a user's fingers.

7. The needle assembly of claim 6, wherein the handle extension includes one or more arcuate surfaces.

8. The needle assembly of claim 1, further comprising a hub fixedly attached to the base end of the needle for attaching the needle to the base.

9. The needle assembly of claim 8, wherein the base and the hub include corresponding engaging surfaces for attachment therebetween.

10. The needle assembly of claim 9, wherein the base and the hub include corresponding engaging surfaces for threaded engagement therebetween.

11. The needle assembly of claim 1, wherein the sleeve comprises one or more moldable plastics.

12. The needle assembly of claim 11, wherein the plastics are one or more selected from the group consisting of polyethylenes, polypropylenes, polyamides, polyesters and fluorinated polyethylenes.

13. A shieldable, single-use needle assembly for administering a unit dose of a vaccine comprising:
(a) a sleeve including a tubular wall defining an internal sleeve opening extending between a proximal end and an opposed distal end, said sleeve including a guide slot extending through the tubular wall into the internal sleeve opening, said guide slot including a longitudinal extent extending longitudinally along the sleeve, a proximal limit extent disposed at a first end of the guide slot adjacent said proximal end of said tubular wall and a distal limit extent disposed at a second end of the guide slot adjacent said distal end of said tubular wall, said proximal and distal limit extents extending along a circumferential surface of the tubular wall;
(b) a base including a tab disposed on an outer surface thereof, said base slidably engaged in said internal sleeve opening with said tab extending through said guide slot, said base including a handle extension extending beyond the proximal end of the sleeve; and
(c) a unit dose needle having a base end attached to the base and an opposed prong end configured to hold a unit dose of a vaccine;
wherein the shield is slidably movable about the needle assembly between a first position in which the needle extends from the distal end of the sleeve and a second position in which the needle is contained within the internal sleeve opening, the tab and the guide slot providing for guiding action through the guide slot between the proximal limit extent and the distal limit extent, thereby causing the shield to move about the needle assembly between the first position and the second position.

14. The needle assembly of claim 13, wherein the proximal and distal limit extents fix the position of the needle between the first position and the second position, respectively.

15. The needle assembly of claim 13, wherein the handle extension includes an internal luer taper to engage a medical device.

16. The needle assembly of claim 13, wherein the handle extension includes a profile for accommodating a user's fingers.

17. The needle assembly of claim 17, wherein the handle extension includes one or more arcuate surfaces.

18. A shieldable single-use needle assembly for administering a unit dose of a vaccine comprising:
(a) a unit dose needle having a prong end configured to hold a unit dose of a vaccine;
(b) a hub attached to the unit dose needle opposite the prong end, the hub including a tapered luer;
(c) a base including a tab disposed on an outer surface thereof, said base threadably attached to the tapered luer of the hub; and
(d) a hollow sleeve extending about said base, said sleeve including a tubular wall extending between a proximal end and an opposed distal end and defining an internal sleeve opening, said sleeve including a guide slot extending through the tubular wall into the internal sleeve opening, said guide slot including a longitudinal extent extending longitudinally along the sleeve, a proximal limit extent disposed at a first end of the guide slot adjacent said proximal end of said tubular wall and a distal limit extent disposed at a second end of the guide slot adjacent said distal end of said tubular wall, said proximal and distal limit extents extending along a circumferential surface of the tubular wall;
wherein said tab extends through and beyond said guide slot such that movement of the tab through the guide slot between the proximal limit extent and the distal limit extent provides a guiding action that enables an extension and a retraction movement of the needle between an extended position with the needle extending from the distal end of the sleeve and a retracted position with the needle contained within the internal sleeve opening, and wherein the proximal and distal limit extents fix the position of the needle between the extended position and the retracted position.

19. A shieldable needle assembly for administering a unit dose of a vaccine comprising:
a) a unit dose needle assembly comprising:
i) a unit dose needle including a prong end configured to hold a unit dose of a vaccine, and
ii) a hub fixedly attached to the unit dose needle opposite the prong end; and
b) a shield assembly attached to said unit dose needle assembly, said shield assembly comprising:
i) a sleeve including a tubular wall defining an internal sleeve opening extending between a proximal end and an opposed distal end, said sleeve including a guide slot extending through the tubular wall into the internal sleeve opening; and
ii) a base including a tab disposed on an outer surface thereof, said base contained within said internal sleeve opening with said tab extending through and beyond said guide slot;
wherein the hub of the unit dose needle assembly and the base of the shield assembly include corresponding structure for attachment therebetween, and wherein the sleeve and the base are movable with respect to each other, thereby enabling an extension and a retraction movement of the needle between an extended position with the needle extending from the distal end of the sleeve and a retracted position with the needle contained within the internal sleeve opening.

20. The needle assembly of claim 19, wherein the hub and the base include corresponding threaded surfaces for threaded engagement therebetween.

21. A shieldable, single-use needle assembly for administering a unit dose of a vaccine comprising:
(a) a base;
(b) a unit dose needle having a base end attached to the base and an opposed prong end configured to hold a unit dose of a vaccine;
(c) a sleeve including a tubular wall defining an internal sleeve opening extending between a proximal end and an opposed distal end, said sleeve axially coacting with said base between a prong exposed position and a prong encapsulated position; and
(d) a guiding element interengaged between said sleeve and said base providing limited axial movement of said sleeve with respect to said base.

22. The needle assembly of claim 21, wherein said guiding element is a tab extending from said base into a slot in said sleeve.

23. The needle assembly of claim 22, wherein said slot extends through said sleeve and said tab is a means to manually move said base unit dose needle into said sleeve.

24. The needle assembly of claim 21, wherein said base includes a handle extension extending beyond the proximal end of said sleeve.

25. A shieldable, single-use needle assembly for administering a unit dose of a vaccine comprising:
(a) a base;
(b) a unit dose needle having an axis, a base end attached to the base and an opposed prong end configured to hold a unit dose of a vaccine;
(c) a tip guard slidable along said unit dose needle axis from a first position adjacent said base to a second position encapsulating said unit dose needle prong end; and
(d) a tether element having a base end integral with said base, a guard end integral with said tip guard, a hinge between said base end and said guard end, a forward member delimited by said guard end and said hinge flexibly pivotable with respect to said guard, a rearward end delimited by said base end and said hinge flexibly pivotable with respect to said base, and an angle between said forward and rearward members wherein forward movement of said hinge enlarges said angle and provides direct forward movement of said guard with respect to said base.

26. The single-use needle assembly of claim 25, wherein said tip guard has a lockout leg preventing a return movement of said tip guard with respect to said base once said lockout leg moves past said prong end of said unit dose needle.
